Europäisches Patentamt

European Patent Office

Office européen des brevets

(19)

(11) Publication number: **0 018 695**
**B1**

(12) **EUROPEAN PATENT SPECIFICATION**

(45) Date of publication of patent specification: **01.09.82**

(51) Int. Cl.³: **C 07 D 251/60**

(21) Application number: **80200403.6**

(22) Date of filing: **01.05.80**

(54) Method for the preparation of melamine.

(30) Priority: **03.05.79 NL 7903473**

(43) Date of publication of application:
**12.11.80 Bulletin 80/23**

(45) Publication of the grant of the patent:
**01.09.82 Bulletin 82/35**

(84) Designated Contracting States:
**AT BE DE FR GB IT NL SE**

(56) References cited:
**GB - A - 1 187 808**
**GB - A - 1 237 780**
**GB - A - 1 282 298**

(73) Proprietor: **STAMICARBON B.V.**
**Postbus 10**
**NL-6160 MC Geleen (NL)**

(72) Inventor: **Van Hardeveld, Rudolf**
**Vondellaan 15**
**NL-6165 EB Geleen (NL)**
Inventor: **Moreau, Dominique Jean J.S.M.**
**Sartonlaan 2**
**NL-6132 BD Sittard (NL)**
Inventor: **Bruls, Pierre Gerard Marie Bernard**
**Schout Boutenstraat 21**
**NL-6121 HC Born (NL)**
Inventor: **Van Hinsberg, Johannes Gerardus**
**Burg. Visschersstraat 21**
**NL-6235 EA Ulestraten (NL)**

(74) Representative: **Hatzmann, Marinus Jan et al,**
**OCTROOIBUREAU DSM P.O.Box 9**
**NL-6160 MA Geleen (NL)**

Courier Press, Leamington Spa, England.

## Method for the preparation of melamine

The invention relates to a method for the preparation of melamine by converting urea and/or thermal decomposition products thereof in a fluid bed of catalytically active material and desublimating and separating melamine, by cooling, from the gas mixture formed in that process, with formation of an off gas mixture consisting mainly of $NH_3$ and $CO_2$.

From Hydrocarbon Processing, September 1969, pp, 184—186, it is known to prepare melamine in such a manner at atmospheric pressure and to subsequently separate it from the reaction gases formed by means of a so-called 'dry catch'.

According to this method, the off gas mixture is washed after separation of the melamine from it, with liquid urea.

The British patent specification 1,187,808 describes the cooling of the off gas mixture obtained after the separation of melamine therefrom, with a ureamelt containing an ammonium and/or a guanidine salt.

This washing with liquid urea is necessary among others, to prevent, in the recirculation of the off gas mixture, a cumulation of impurities in the circulating fluidizing gas. As, however, the temperature of the liquid urea must not be too high, in order to prevent all kinds of secondary reactions, a large amount of heat of a low temperature level must be removed, in the washing column, with cooling water.

Moreover, a part of the liquid urea is taken along in the off gas mixture in the form of drops. These urea drops cause clogging in lines and equipment and must therefore be removed from the off gas mixture, with all problems involved, such as incrustation.

The British patent specification 1,237,780 describes a method for the preparation of melamine in the presence of added ammonia, cooling the melamine containing gases with $NH_3$, separating the melamine from these gases, and further cooling the obtained off gas mixture.

In the process according to this patent specification, carbon dioxide is separated from the off gas mixture as ammonium carbamate. The resulting ammonia is recycled to the melamine preparation.

This separation of carbon dioxide is energetically unfavourable.

The purpose of the invention is to provide a method for the preparation of melamine in which these problems do not occur.

The invention is characterized in that, optionally after filtration to remove entrained dust, the larger part of the off gas mixture is used, after compression to compensate for the loss of pressure, directly as fluidizing gas for the bed of catalytically active material.

Specifically, 50—90% of the off gas mixture is used as fluidizing gas.

It is particularly surprising that it is possible, without further treatment, to use the off gas mixture from a dry capture as fluidizing gas without any cumulation of impurities in the circulating gas mixture, so that very pure melamine can be obtained.

In a preferred embodiment of the method according to the invention the partial pressure of $NH_3$ in the fluid bed of catalytically active material is over 100 kPa, more specifically between 350—2500 kPa. The fact is that it has been found that, particularly at elevated partial pressures of $NH_3$, i.e. more than 70 kPa, preferably more than 100 kPa but specifically more than 350 kPa, hardly any or no impurities are formed in the form of melam, melem, melon, ammeline and ammelide so that the purification steps to remove these compounds are superfluous. On the other hand, these pressures are not so high as to make expensive equipment necessary.

A process of this kind is described in the copending application 80 200405.1, of the same date, publication number 18.696.

The separation of melamine from the gases coming from the reactor can be effected by direct or indirect cooling. For instance, cold $NH_3$ gas or gas mixtures of $NH_3$ and $CO_2$ can be mixed with the reaction gases.

Preference, however, is given to the application of a desublimation of melamine in one or more fluid beds of melamine particles with application of indirect cooling. This indirect cooling will preferably be effected with cooling water, in which process, by running the cooling water counter current to the melamine reaction gases, high-quality steam is formed.

The advantage of this method is that relatively large melamine particles are obtained with a relatively narrow particle size distribution. Moreover, the melamine obtained in this manner is free flowing, so that is can be conveyed in bulk, which is not the case when direct cooling is applied.

The temperature in the desublimation step is preferably between 180 and 250°C. The pressure at which the desublimation is effected will generally be a little lower than the pressure in the reactor in consequence of loss of pressure in lines and equipment.

As the desublimation step often occurs at higher than atmospheric pressure, a special system is necessary to remove the melamine powder from the desublimation equipment. This can, for instance, be done with a cyclone, a rotating valve, or a device as described in the Dutch patent application 7600277 laid open to public inspection.

The method according to the invention has the great advantage that relatively little equipment is necessary, while the consumption of energy compared with the known method is substantially lower. In the method according to the invention it is possible to convert the heat content of the reaction gases, that is the desublimation heat of the melamine and the so-called sensible heat direct into high-quality steam. Moreover, maximum use is made of the heat content of the off gases, freed of melamine, for the conversion of urea into melamine by passing these gases without further treatment into the melamine reactor. In applying the known method, on the other hand, this heat content is discharged at such a low temperature level it cannot or hardly be put to any useful effect.

If, before being used a fluidizing gas, the off gases freed of melamine are compressed to compensate for loss of pressure in the reactor and the desublimator, a heat exchange is preferably applied between the gas before and after compression. Such a method has the great advantage that extremely fine melamine particles, which despite the dust catching cyclone of the desublimation step, are yet present in the off gases, will sublimate again, so that hardly any or no dust will enter the compressor. The fact is that, in connection with wear, it is undesirable for dust to enter a compressor.

If, moreover, the off gases contain catalyst dust as well, it is desirable yet to filter it off. This will then preferably be effected after the heat exchange, but before the compression.

A part of the off gases freed of melamine must be removed, because in the conversion of urea into melamine six molecules of ammonia and three molecules of $CO_2$ per molecule of melamine are formed. In general 10—40% will be removed. For the processing of this off gas flow various methods can be applied.

It is possible to absorb the off gas flow, which mainly consists of ammonia and $CO_2$ in a molecular ratio of two, in water or an aqueous solution and, optionally after concentration, to feed it as a carbamate flow to a urea synthesis.

Other possibilities are compression of the gas mixture to urea synthesis pressure or processing into an ammonia-containing fertilizer.

Finally, it is also possible to separate the gas mixture by means of an $NH_3/CO_2$ separation into the separate components (Dutch patent applications 6706135, 7612163 and 7404403).

The preparation of melamine is effected as known in the art, for instance as described in the Dutch patent applications 7700509 and 7700553 laid open to public inspection.

As catalyst in the fluid bed one of the known catalysts can be used, such as aluminium oxide, aluminium oxide on silicon oxide, silicon oxide, titanium oxide, zirconium oxide, boron phosphate or aluminium phosphate or a mixture of 2 or more of these catalysts. The expression catalyst or catalytically active material is understood here to mean any material promoting, under the reaction conditions applied, the conversion of urea into melamine. The temperature at which the melamine is formed from urea is generally more than 325°C. In general this temperature will not be above 425°C, more specifically preference is given to temperatures of between 370 and 400°C. The temperature applied depends, in part, on the pressure in the reactor, at higher pressure a higher temperature is preferably applied.

The invention is now elucidated by means of a figure, but is not limited to it. This figure shows a diagrammatic embodiment of the method according to the invention.

In reactor 1 urea is atomized, through sprayers 2, 3, in a fluid bed of catalyst particles. This bed is fluidized by means of a gas mixture supplied through fluidizing gas supply 4 and gas distributor plate 5. In the fluid bed the desired temperature is maintained by means of heat exchanger tubes 6, which have been shown here diagrammatically. The melamine-containing reactor gases are fed, through cyclone 7 and line 8, to desublimator 9. In this desublimator 9, which is in the form of one or more fluid beds of melamine particles, melamine is desublimated.

Through heat exchanger tubes 10, the heat released in this process is removed and converted into high pressure steam.

At the bottom of desublimator 9 solid melamine particles are removed through line 11. In separator 12 melamine is separated from the gases. The gases are returned, through line 13, to the desublimator 9. Through line 14, and after pressure reduction via valve 15, the melamine is removed.

Through cyclone 16 and line 17 the off gas mixture, freed of melamine, is removed. A part of the off gas mixture is removed through line 18, for instance to a urea synthesis, an $NH_3$—$CO_2$ separation or a fertilizer preparation. The remaining part is recirculated, through line 19, compressor 20, line 21 and heat exchanger 22, to the reactor 1.

The invention will now be elucidated by means of an example but is not restricted to it.

EXAMPLE

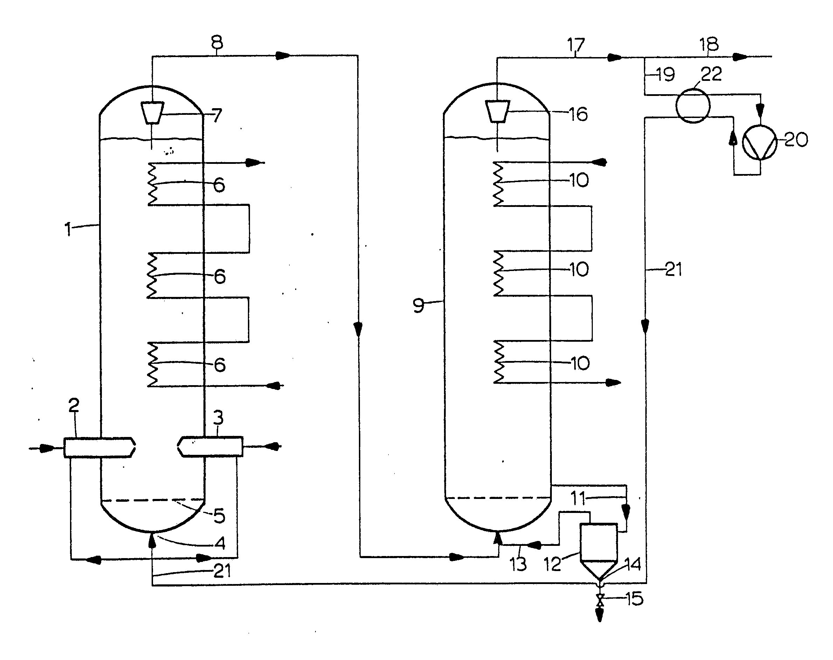

The melamine preparation was performed in an installation as described in the figure.

To the melamine reactor, operating at a pressure of 1000 kPa and a temperature of 375°C, 20,000 kg of urea and 30,000 kg of gaseous $NH_3$ and $CO_2$ were fed per hour.

The reaction gases, containing melamine vapours, were fed to desublimator 9, which also operated at a pressure of 900 kPa. This desublimator contained a fluid bed with melamine particles. per hour about 7000 kg of melamine with good free-flowing properties and a purity of 99.9% was discharged from the desublimator.

Of the off gas mixture, largely freed of melamine, 80% was recirculated to the melamine reactor, after compression to compensate for the fall in pressure across the reactor and the desublimator, and heat exchange. The remaining 20% was fed to an NH$_3$/CO$_2$ separation.

## Claims

1. Method for the preparation of melamine by converting urea and/or thermal decomposition products thereof in a fluid bed of catalytically active material and desublimating and separating melamine, by cooling, from the gas mixture formed in that process, with formation of an off gas mixture consisting mainly of NH$_3$ and CO$_2$, characterized in that, optionally after filtration to remove entrained dust, the larger part of the off gas mixture is used, after compression to compensate for the loss of pressure, directly as fluidizing gas for the bed of catalytically active material.

2. Method according to claim 1, characterized in that 50—90% of the off gas mixture is used as fluidizing gas.

3. Method according to claim 1 or 2, characterized in that the melamine is separated in one or more fluid beds of melamine particles.

4. Method according to claim 3, characterized in that the melamine is separated in one or more fluid beds of melamine particles with indirect cooling by means of cooling water, in which process steam is formed.

5. Method according to claim 4, characterized in that the cooling water is run counter current to the melamine-containing reaction gases.

6. Method according to one of the claims 1—5, characterized in that the partial pressure of NH$_3$ in the fluid bed of catalytically active material is over 70 kPa.

7. Method according to claim 6, characterized in that the partial pressure of NH$_3$ in the fluid bed of catalytically active material is between 350 and 2500 kPa.

8. Method according to one of the claims 1—7, characterized in that the off gas mixture to be compressed, substantially freed of melamine is subjected to an exchange of heat with the compressed gas.

## Revendications

1. Procédé de préparation de mélamine en convertissant de l'urée et/ou des produits de décomposition thermique de celle-ci dans un lit fluidisé de matériau catalytiquement actif, en désublimant et en séparant la mélamine par refroidissement à partir du mélange de gaz formé lors de ce procédé, avec formation d'un mélange de gas résiduaires composé essentiellement de NH$_3$ et de CO$_2$, caractérisé en ce que la majeure partie du mélange de gaz résiduaires — éventuellement après filtration pour éliminer la poussière entraînée et après compression pour compenser la perte de pression — est directement utilisée comme gaz de fluidisation pour le lit de matériau catalytiquement actif.

2. Procédé selon la revendication 1, caractérisé en ce que 50—90% du mélange de gaz résiduaires sont utilisés comme gaz de fluidisation.

3. Procédé selon l'une des revendications 1 à 2 caractérise en ce que la mélamine est séparée dans un ou plusieurs lits fluidisés de particules de mélamine.

4. Procédé selon la revendication, 3 caractérisé en ce qu'on sépare la mélamine dans un ou plusieurs lits fluidisés de particules de mélamine, sous refroidissement indirect à l'aide d'eau de refroidissement, et avec production de vapeur.

5. Procédé selon la revendication 4, caractérisé en ce que l'eau de refroidissement s'écoule en contre-courant avec les gaz réactionnels contenant de la mélamine.

6. Procédé selon l'une des revendications 1 à 5, caractérise en ce que la pression partielle du NH$_3$ dans le lit fluidisé de matériau catalytiquement actif est supérieure à 70 kPa.

7. Procédé selon la revendication 6, caractérisé en ce que la pression partielle du NH$_3$ dans le lit fluidisé de matériau cataiytiquement actif se situe entre 350 et 2500 kPa.

8. Procédé selon l'une des revendications 1 à 7, caractérisé en ce que le mélange de gaz résiduaries à comprimer, qui est essentiellement exempt de mélamine, est soumis à un échange de chaleur avec le gaz comprimé.

## Patentansprüche

1. Verfahren zue Herstellung von Melamin, indem man Harnstoff und/oder thermische Zersetzungsprodukte dieses Stoffes in einem Fliessbett aus katalytisch wirksamer Masse umsetzt und Melamin aus dem dabei anfallenden Gasgemisch durch Abkühlung entsublimiert und abscheidet unter Bildung eines im wesentlichen aus NH$_3$ und CO$_2$ bestehenden Abgasgemisches, dadurch gekennzeichnet, das man den grössten Teil des Abgasgemisches, ggf, nach Filtrierung zwecks Entfernung von mitgeführten Staubeilchen und nach Verdichtung zum Ausgleich von Druckverlusten, direkt als Fliessgass für das aus katalytisch wirksamer Masse bestehende Bett verwendet.

2. Verfahren nach Anspruch 1, dadurch gekennzeichnet, dass das Abgasgemisch zu 50—90% als Fliessgas benutzt wird.

3. Verfahren nach Anspruch 1 oder 2, dadurch gekennzeichnet, dass man das Melamin in einem oder mehreren aus Melaminteilchen bestehenden Fliessbetten abscheidet.

4. Verfahren nach Anspruch 3, dadurch gekennzeichnet, dass das Melamin in einem oder mehreren aus Melaminteilchen bestehenden Fliessbetten unter indirekter Kühlung mit Hilfe von Kühlwasser mit Bildung von Wasserdampf abgeschieden wird.

5. Verfahren nach Anspruch 4, dadurch gekennzeichnet, dass man das Kühlwasser im Gegenstrom zu den melaminhaltigen Reaktionsgassen leitet.

6. Verfahren nach den Ansprüche 1—5, dadurch gekennzeichnet, dass der Partialdruck von NH₃ im Fliessbett von katalytisch wirksamer Masse mehr als 70 kPa beträgt.

7. Verfahren nach Anspruch 6, dadurch gekennzeichnet dass der Partialdruck von NH₃ im Fliessbett von katalytischen wirksamer Masse zwischen 350 und 2500 kPa liegt.

8. Verfahren nach den Ansprüche 1—7, dadurch gekennzeichnet, dass man das zu verdichtende nunmehr melaminfreie Abgasgemisch im Wärmeaustausch mit dem zu verdichtenden Gas treten lässt.

0 018 695